# EUROPÄISCHE PATENTSCHRIFT

(11) **EP 3 979 963 B1**
(45) Veröffentlichungstag und Bekanntmachung des Hinweises auf die Patenterteilung: **08.05.2024**
(21) Anmeldenummer: 20727963.9
(22) Anmeldetag: 19.05.2020
(51) Int. Cl.: A61F 13/02, A61F 13/15

(54) **HAUTAUFLAGE**
DERMAL PATCH
TIMBRE TRANSDERMIQUE

(30) Priorität: 04.06.2019 DE 102019115005
(43) Veröffentlichungstag der Anmeldung: 13.04.2022
(73) Patentinhaber: Carl Freudenberg KG, 69469 Weinheim (DE)
(72) Erfinder: LANG, Birthe, 69469 Weinheim (DE); HOFBAUER, Thomas, 69493 Hirschberg (DE); SCHLESSELMANN, Bernd, 69469 Weinheim (DE); PEHR, Marc, 69514 Laudenbach (DE)
(86) Internationale Anmeldenummer: PCT/EP2020/063959
(87) Internationale Veröffentlichungsnummer: WO 2020/244924

(56) Entgegenhaltungen:
- WO-A1-2015/096230
- US-A1- 2013 116 645
- US-A1- 2014 249 495
- US-A1- 2018 008 476
- US-B2- 8 357 256

## Beschreibung

### Technisches Gebiet

Druckgeschwüre (oder Dekubitus Wunden) stellen seit langem ein signifikantes Problem sowohl im klinischen als auch im ambulanten und stationären Pflegebereich dar. Für Patienten sind solche Wunden mit starken Schmerzen, reduzierter Lebensqualität und erhöhter Morbidität verbunden. Für das Gesundheitswesen entstehen hohe Behandlungskosten insbesondere durch verlängerte Krankenhausaufenthalte und intensiven Pflegebedarf. In vielen Ländern werden im Krankenhaus und in der Pflegeeinrichtung auftretende Druckgeschwüre bereits als Qualitätsmerkmal verwendet, d.h. eine hohe Inzidenzrate indiziert geringe Qualität in der Pflege.

Druckgeschwüre entstehen durch eine Kombination von hohem oder ungleichmäßigem Druck, Scherkräften und Reibung bei temporär oder permanent immobilisierten Patienten. Der Ursprung dieser Geschwüre liegt meist im tieferliegenden Gewebe, insbesondere über kantigen Knochenvorsprüngen wie Ferse oder Sakralregion. Wird das Gewebe durch Druck und Scherkräfte verformt, setzt eine Entzündungsreaktion des Körpers ein, die letztendlich zum Absterben von Zellen und Schädigungen im tieferliegenden Gewebe führt. Diese Schädigungen weiten sich in einer Kettenreaktion auf höherliegende Gewebeschichten aus. Oftmals sind sie erst nach mehreren Tagen an der Hautoberfläche sichtbar. Auch das Mikroklima auf der Haut trägt zur Bildung von Druckgeschwüren bei. Bei exzessiver Feuchtigkeit auf der Haut (bspw. Schweiß oder Urin) kann erhöhte Reibung auftreten und zur Degradation der Haut führen. Exzessive Trockenheit der Haut kann wiederum auch zu einer brüchigen Haut führen, die schnell verletzt.

Zur Prophylaxe von Dekubiti werden standardmäßig verschiedene Maßnahmen angewendet. Diese beinhalten die Repositionierung von Patienten, die Verwendung druckentlastender Matratzen und vorbeugende Hautpflege. Unter anderem kann die prophylaktische Verwendung von Wundauflagen und regelmäßige Pflege und Inspektion der Haut signifikant zur Verhinderung von Druckgeschwüren beitragen.

Eine Wundauflage, die primär zur Heilung eingesetzt wird, aber auch zur Prävention beschreibt die EP1156838B1. Die Wundauflage besteht aus einer hydrophilen Schaumschicht, die zur Haut zugewandten Seite mit einer adhäsiven Schicht ausgerüstet ist und auf der gegenüberliegenden Seite mit einer absorbierenden Lage. Nachteilig an der Verwendung dieser Auflage ist, dass die Auflage primär zur Heilung, d.h. zur Absorption und Retention von Wundexsudat konfiguriert, und somit die Funktion nicht für die Prävention optimiert ist. D.h. es ist keine Schicht vorhanden, die zur Druckverteilung optimiert ist.

In US 7182085 A1 wird eine Wundauflage beschrieben, bestehend aus einem absorbierenden und einem nicht absorbierenden druckverteilenden Teil, die gezielt Wundexsudat absorbiert und zugleich eine Druckverteilung von statischen und dynamischen Druckeinwirkungen gewährleistet. Nachteilig an der Wundauflage ist, dass sie einen sehr komplizierten Aufbau aufweist, da sie sowohl den Anforderungen zur Absorption als auch zur Druckverteilung entsprechen muss. Außerdem ist sie nicht zuschneidbar.

US 9877872 B2 beschreibt ebenfalls eine Wundauflage zur Heilung, die zusätzlich zu einer Absorptionsschicht mit einer designierten Druckverteilungslage in Form eines Abstandgewirkes/ -gestricks oder -gewebes zur Dekubitus Prävention ausgestattet ist. Nachteilig ist hier die Gestaltung in Form eines sogenannten Island Dressings, welches zum einen keine Wiederverwendung der Auflage nach einer Hautinspektion ermöglicht, da die Filmränder leicht verkleben und zum anderen nicht für alle Körperregionen optimiert sind. Hinzu kommt, dass die für Mikroorganismen undurchlässige Filmseite, welche bei einem Einsatz auf intakter Haut nicht benötigt wird, den Feuchtigkeitsabtransport limitiert.

Eine Wundauflage mit zur Dekubitus Prävention optimierten Eigenschaften in der Sakralregion beschreibt US 20180008476 A1. Die Wundauflage weist zudem Laschen an der Auflage auf, die ein einfaches Ablösen und Wiederbefestigen an der Haut und somit eine Hautinspektion ermöglichen. Des Weiteren zeichnet sich die Auflage durch unterschiedliche Zugfestigkeiten in x und y Richtung aus. In y-Richtung liegt eine höhere Zugfestigkeit vor, um beim Hinabgleiten des Patienten im Bett Zellen vor Deformation zu schützen. In x-Richtung ist die Auflage durch eine höhere Dehnbarkeit charakterisiert um entstehende Kräfte beim Wenden des Patienten zu kompensieren. Nachteilig ist hier, dass die Form nur für die Sakralregion geeignet optimiert ist und nicht durch bspw. Zuschneiden, auch in anderen Körperregionen effizient verwendet werden kann, da das Zug- Dehnungsverhalten auf die Region abgestimmt ist. Auch ist die Form und Größe nicht für einzelne Patienten individualisierbar.

Eine adhäsive in der Form teilweise anpassbare Wundauflage für die Sakralregion wird beispielsweise in der EP1320343B1 beschrieben. Aufgrund der "Island-Dressing" Form ist auch hier ein Zuschneiden nicht möglich.

WO 2017095460 A1 beschreibt eine hydrophile polymere Matrix mit geschlossenen Zellen zum direkten Hautkontakt, um Druck zu verteilen und Feuchtigkeit zu regulieren. Die Auflage besteht aus einer Schicht und kann daher zugeschnitten, d.h. patientenindividualisiert werden. Die Wundauflage zielt auf die Gewährleistung von Heilung, d.h. Absorption und Prävention kombiniert in einer Schicht. Dies führt zwangsläufig zur Kompromittierung der druckverteilenden Eigenschaften zu Gunsten einer erhöhten Absorptionskapazität, die für einen rein prophylaktischen Einsatz jedoch nicht notwendig ist.

Des Weiteren sind Auflagen bekannt, die alleinig auf die Prävention abzielen. EP 3162330 A1 beschreibt eine anatomisch geformte Auflage für den Schutz der Sakralregion, bestehend aus einer Feuchtigkeitsbarriere-Schicht und einer zur Haut zugewandten verbindenden Schicht. Zwar kann ein gezieltes Feuchtigkeitsmanagement auf der Haut die Bildung von Druckgeschwüren reduzieren, ist jedoch alleinig nicht ausreichend, da keine gerichtete Druckverteilung stattfindet. Die Form der Auflage eignet sich zudem nur für Sakralregion. Ein Zuschneiden oder eine Adaption für andere Körperregionen ist nicht effizient möglich.

US 20170087002 A1 beschreibt eine Druckverteilungsauflage bestehend aus einer Druckverteilungslage vorzugsweise einer zellulären Schaumstruktur, einem dünnen Film und einer Ablöselage zur Prävention von Druckgeschwüren insbesondere im Gesicht bei Anwendungen von medizinischem Equipment. Als Druckverteilungsschicht wird ein Polyurethanschaum mit definierter Dichte verwendet, der auf einem dünnen hydrophilen Film mit einem Release Liner aufgebracht ist. Die Auflage ist zuschneidbar und kann an den Patienten angepasst werden. Nachteilig ist hier, dass die Druckverteilung nur durch den Schaum stattfindet. Es ist davon auszugehen, dass die Zellen im Schaum unter starkem Druck, insbesondere in stark belasteten Bereichen wie der Sakralregion kollabieren und keinen ausreichenden Schutz bieten. Dies trifft insbesondere im feuchten Zustand des Schaumes zu. Ferner ist fraglich, ob ein dünner hydrophiler Film genug Kapazität besitzt, um beispielsweise Schweiß schnell von der Haut abtransportieren zu können.

Die WO 2017039668 A1 beschreibt ein Produkt zur Prävention von Druckgeschwüren umfassend ein Substrat mit einer inneren Substratoberfläche, die einen Substratbereich und eine gegenüberliegende äußere Substratoberfläche aufweist; wobei die gegenüberliegende äußere Substratoberfläche einen dynamischen Reibungskoeffizienten im Bereich von 0,1 bis 0,6 aufweist; eine Verbindungsschicht, die auf der inneren Substratoberfläche angeordnet ist; eine Polsterschicht mit einer dem Körper zugewandten Oberfläche, die einen Polsterschichtbereich und eine gegenüberliegende, dem Substrat zugewandte Oberfläche aufweist; und eine Adhäsivschicht, die auf der dem Körper zugewandten Oberfläche angeordnet ist; wobei die Polsterschicht zwischen der Haftschicht und der Adhäsivschicht angeordnet ist und wobei das Produkt zur Prävention von Druckgeschwüren eine Wasserdampfdurchlässigkeit im Bereich von 2.400 g / m 2 * Tag bis 10.000 g / m 2 * Tag aufweist. Als Polsterlage kann ein Schaum oder Vliesstoff, beispielsweise ein gewellter Vliesstoff eingesetzt werden. Nachteilig an dem Produkt ist, dass die Polsterschicht lediglich durch die Adhäsivschicht von der Haut getrennt wird. Bei einer dünnen Adhäsivschicht können bei Verwendung eines Vliesstoffs Hautirritationen durch die Adhäsivschicht durchdringende Fasern verursacht werden. Bei einer dicken Adhäsivschicht wird wiederum die Wasserdampfdurchlässigkeit verschlechtert.

Aus der WO 2018/007093 A1 ist eine Hautauflage bekannt umfassend eine offenzellige Schaumlage und eine darauf angeordnete, für den Hautkontakt vorgesehene, adhäsive Schicht, wobei zumindest die der adhäsiven Schicht zugewandte Seite der Schaumlage Makroporen aufweist, deren Kavitäten zumindest anteilig durch eine aus der Schaumlage gebildete Sperrschicht überspannt sind. Die Hautauflage eignet sich hervorragend zur Feuchteregulierung in bestehenden (chronischen) Wunden, insbesondere zur Erzeugung eines feuchten Wundklimas und mithin zur feuchten Wundbehandlung, insbesondere von chronischen Wunden. Für die Verwendung zur Verhinderung von Druckgeschwüren ist die Hautauflage jedoch nicht optimiert.

Die WO 2015/096230 A1 beschreibt einen schäumenden Verbundwundverband, umfassend eine Wasserabsorptionsschicht und eine Wasserrückhalteschicht, die die Wasserabsorptionsschicht bedeckt; eine Übergangsschicht, die zwischen der Wasserabsorptionsschicht und der Wasserrückhalteschicht angeordnet ist; eine wasserdichte Schicht, die die Wasserrückhalteschicht bedeckt; eine Polyurethanfilmschicht, die unterhalb der Wasserabsorptionsschicht angeordnet ist.

US 2013/116645 A1 beschreibt ein Schaumstoffband mit einer Klebeschicht zur Positionierung auf einer Gewebeoberfläche, einer porösen Schicht mit einer Vielzahl von Poren, die geeignet sind, Flüssigkeit zu absorbieren, einer atmungsaktiven Schicht, die so konfiguriert ist, dass sie den Durchgang von Dampf ermöglicht, und einer optionalen Ablöseschicht. Das Schaumstoffband kann als Wundverband oder in einem Kompressionsverbandsystem verwendet werden.

US 2014/249495 A1 beschreibt einen Wundverband, umfassend:
eine Wundkontaktschicht; eine absorbierende Schicht zum Absorbieren von Wundexsudat, wobei die absorbierende Schicht oberhalb der Wundkontaktschicht angeordnet ist; ein Verdunkelungselement zum zumindest teilweisen Verdunkeln einer Sicht auf Wundexsudat, das von der absorbierenden Schicht im Gebrauch absorbiert wird, wobei das Verdunkelungselement über der absorbierenden Schicht angeordnet ist; und eine Abdeckschicht, die über dem Verdunkelungselement angeordnet ist.

Kommerziell verfügbare Auflagen sind beispielsweise Mepilex^{®} Border (Mölnlycke^{®} Healthcare) und Allevyn Life (Smith & Nephew). Beide verfügen über einen mit Silikon beschichteten Schaum zur Adhäsion auf der Haut, sowie eine einen Superabsorber enthaltende Vliesschicht. Zusätzlich verfügt das Allevyn Life Produkt über eine druckverteilende Lage in Form eines Abstandgewirkes. Nachteilig an beiden Produkten ist, dass sie eine vergleichsweise dicke Silikonschicht (zwischen 200 µm und 300 µm) und eine signifikante Menge an Superabsorber aufweisen, was die Kosten der Produkte erhöht.

Aufgabe der vorliegenden Erfindung ist es, eine Hautauflage bereitzustellen, die für ihren Einsatz zur Verhinderung von Druckgeschwüren optimale Eigenschaften aufweist. So soll die Hautauflage im Gewebe auftretende Druckspannungen effektiv reduzieren können sowie zur Regulierung der Feuchtigkeit in Hautnähe geeignet sein. Darüber hinaus sollen die oben genannten Nachteile zumindest teilweise ausgeräumt werden.

Diese Aufgabe wird gelöst durch eine Hautauflage umfassend eine Schaumlage und eine unmittelbar darauf angeordnete, für den Hautkontakt vorgesehene, adhäsive Schicht, wobei auf der der adhäsiven Schicht abgewandten Seite der Schaumlage ein textiles Flächengebilde angeordnet ist, wobei das textile Flächengebilde ein Vertikalvliesstoff ist.

Überraschend wurde gefunden, dass die erfindungsgemäße Hautauflage sich hervorragend zur Verhinderung von Druckgeschwüren eignet, da sie effizient im Gewebe auftretende Druckspannungen reduzieren kann. Ohne sich erfindungsgemäß auf einen Mechanismus festzulegen, wird vermutet, dass diese vorteilhaften Eigenschaften der Hautauflage dadurch zu Stande kommen, dass der Vertikalvliesstoff Fasern mit einer zur Fläche der Hautauflage vertikalen Komponente aufweist - im Gegensatz zu Vliesstoffen, die mit herkömmlichen Vliesbildungsverfahren (bspw. Kardieren mit und ohne Querlegung, Ablage aus dem Luftstrom (Airlay und Airlaid) oder Nasslegeverfahren) hergestellt wurden und die überwiegend horizontal abgelegte Fasern aufweisen. Dies führt dazu, dass die Hautauflage aufgrund der vertikal ausgerichteten Fasern besonders gut auftretende Druckbelastungen aufnehmen und verteilen kann, da die vertikal ausgerichteten Fasern einer Komprimierung der Hautauflage entgegenwirken und so die Belastungsfläche des zu schützenden Körperteils erhöht wird. Hierdurch können auftretende Belastungen im Gewebe reduziert werden. Dies gilt insbesondere für Belastungsspitzen und hat zur Folge, dass die Zellen im Gewebe effektiv vor zu hohen Belastungen geschützt und so die zu Dekubitus führenden Mechanismen abgeschwächt werden.

Weiter vorteilhaft ist, dass erfindungsgemäß die Schaum lage eine Barriereschicht zur Haut darstellt und hierdurch verhindern kann, dass Fasern in Hautkontakt kommen und zu Hautirritationen führen. Darüber hinaus können Schaum lagen auf einfache Weise mit einer ebenen Oberfläche gefertigt werden, was - verglichen beispielsweise mit Vlieslagen - das Aufbringen einer adhäsiven Schicht auch in klar definierten Mustern ermöglicht.

Weiter vorteilhaft an der erfindungsgemäßen Hautauflage ist, dass sie, vermutlich aufgrund der Kapillarwirkung ihrer vertikal ausgerichteten Fasern, eine hohe Wasserdampfdurchlässigkeit (MVTR) aufweist. Dies ist vorteilhaft, da so eine exzessive Ansammlung von Feuchtigkeit auf der Haut verhindert werden kann.

Ebenfalls vorteilhaft ist, dass die Hautauflage als Rollenware hergestellt werden kann und hierdurch zuschneidbar ist, d.h. die Form ideal auf den Patienten angepasst werden kann.

Bei einem Vertikalvliesstoff resultiert die vertikale Komponente aus dessen spezifischem Herstellungsprozess. Im Gegensatz zu einem klassischen Vernadelungsverfahren, bei dem in der Regel nur eine lokale vertikale Ausrichtung eines horizontalen Faserflors in den Einstichkanälen der Nadeln erzielt wird, wird bei der Herstellung eines Vertikalvliesstoffs in der Regel der gesamte gebildete Faserflor in vertikaler Richtung gefaltet und so vertikal zur Faserflorebene ausgerichtet wird. Der Winkel des gefalteten Faserflores zur Ebene des noch nicht gefalteten Flores beträgt vorzugsweise mindestens 20°, noch bevorzugter mindestens 60°, noch bevorzugter etwa 90° und/oder mindestens 90°. Die Fasern beziehungsweise der Flor müssen dabei nicht einer einzigen Gerade beziehungsweise Ebene folgen, sondern können beispielsweise gebogen sein oder einem Zick Zack Verlauf folgen. Beispielhafte, erfindungsgemäß geeignete Herstellungsverfahren für Vertikalvliesstoffe sind in den Dokumenten US 2016/0244895 A1 (V-Lap Verfahren), US 8,357,256 B2 (Wavemaker Santex Verfahren) und CN 104805597 (Anyou-Verfahren) zu finden.

Der Vertikalvliesstoff kann die verschiedensten Matrixfasern enthalten. Unter Matrixfasern sind erfindungsgemäß Fasern zu verstehen, die im Vertikalvliesstoff nicht, oder nur zu einem geringen Teil thermisch verschmolzen vorliegen. Bevorzugt sind die Matrixfasern thermoplastische Matrixfasern, die vorzugsweise einen Schmelzpunkt der am niedrigsten schmelzenden Faserkomponente von über 100°C, beispielsweise von 100 bis 200°C, noch bevorzugter von über 200°C aufweisen. In einer Ausführungsform bestehen die Matrixfasern aus einer Faserkomponente. In einer weiteren bevorzugten Ausführungsform bestehen die Matrixfasern aus mehreren Faserkomponenten. Besonders bevorzugt sind Vollprofilfasern, Multilobalvollprofilfasern, Hohlfasern, Vollprofilbikomponentenfasern (z.B. Kern/Mantel, Side-by-Side). Sofern der Vertikalvliesstoff Bindefasern aufweist, ist es vorteilhaft, wenn der Schmelzpunkt der in der Matrixfaser enthaltenen Faserkomponente mit dem niedrigsten Schmelzpunkt über dem Schmelzpunkt der in der Bindefaser enthaltenen Faserkomponente mit dem höchsten Schmelzpunkt liegt. Vorzugsweise liegt der Schmelzpunkt der Faserkomponente mit dem niedrigsten Schmelzpunkt in der Matrixfaser mindestens 10 °C, besonders bevorzugt mindestens 30 °C, über dem Schmelzpunkt der Faserkomponente mit dem höchsten Schmelzpunkt in der Bindefaser. Geeignete Polymerklassen zur Herstellung der Matrixfasern können unter anderen Polyester, Polyamide, Polyolefine, Polyacrylnitril, Cellulose oder Polyvinylalkohole sein.

Besonders geeignete Matrixasern sind hydrophobe Fasern, d.h. Fasern, die aus Polymeren gefertigt sind, deren Oberflächenenergie niedriger als 50 mJ/m² beträgt. Besonders geeignete hydrophobe Fasern sind Polyester- und/oder Polyolefinfasern. An hydrophoben Fasern ist vorteilhaft, dass sie keine Feuchtigkeit aufnehmen, sondern diese entlang der z-orientierten Struktur vom Körper abtransportieren können. Hierdurch kann eine negative Beeinflussung der Struktureigenschaften des Vliesstoffes, insbesondere der Kompressionseigenschaften, durch die Feuchtigkeit verhindert werden.

Der Anteil der Matrixfasern im Vertikalvliesstoff beträgt vorzugsweise mindestens 20 Gew.%, beispielsweise von 20 bis 100 Gew.%, noch bevorzugter mindestens 25 Gew.%, beispielsweise von 25 bis 80 Gew.%, noch bevorzugter mindestens 30 Gew.%, beispielsweise von 30 bis 70 Gew.%, jeweils bezogen auf das Gesamtgewicht des Vertikalvliesstoffs.

In einer weiteren bevorzugten Ausführungsform der Erfindung weist der Vertikalvliesstoff Bindefasern auf. Bindefasern sind Fasern, die im Vertikalvliesstoff zumindest teilweise verschmolzen sind und hierdurch Bindepunkte zwischen den Fasern schaffen. Vorzugsweise weist mindestens eine schmelzbare Faserkomponente der Bindefaser, insbesondere eine außen angeordnete schmelzbare Faserkomponente, einen Schmelzpunkt auf, der niedriger liegt als der Schmelzpunkt anderer im Vliesstoff enthaltener Faserkomponenten, insbesondere niedriger als der Schmelzpunkt der am niedrigsten schmelzenden Faserkomponente der Matrixfasern.

Weist die Bindefaser mehrere schmelzbare Faserkomponenten auf, so liegt der Schmelzpunkt der am höchsten schmelzenden Faserkomponente der Bindefaser bevorzugt mehr als 10 °C, besonders bevorzugt mehr als 30 °C unter dem Schmelzpunkt der am niedrigsten schmelzenden Faserkomponente der Matrixfasern. Geeignete Bindefasern weisen einen Schmelzpunkt der am höchsten schmelzenden Faserkomponente von unter 250°C, beispielsweise von 100 bis 200°C, noch bevorzugter von unter 180°C, beispielsweise von 100 bis 180°C, insbesondere unter 175°C, beispielsweise von 100 bis 175°C auf.

Bevorzugte schmelzbare Faserkomponenten in Bindefasern sind Polyolefin, Polyester, Polyamid und/oder Gemische hiervon sowie Copolymere wie Ethylen-Vinylacetat-Copolymere. Ebenfalls bevorzugte Bindefasern sind Bikomponentenfasern, insbesondere Bikomponentenfasern, die Polyolefin, Polyester, Ethylen-Vinylacetat-Copolymere, Polybutylenterephtalat, und/oder Gemische hiervon als außen angeordnete Faserkomponente enthalten. Die Bindefasern können unterschiedliche Querschnittsgeometrien wie Vollprofilfaser-, Multilobalvollprofilfaser-, Hohlfaser-, Vollprofilbikomponentenfaser- (z.B. Kern/Mantel, Side-by-Side) geometrien aufweisen. Bevorzugt sind Schmelzbindefasern in Form von Vollprofilfasern.

In einer bevorzugten Ausführungsform der Erfindung sind die Matrixfasern und/oder die Bindefasern gekräuselte Fasern. Vorteilhaft an gekräuselten Fasern ist, dass sie dem textilen Flächengebilde ein verbessertes Rückerholungsvermögen verleihen. Das bedeutet, dass das textile Flächengebilde auch unter starker mechanischer Belastung sein Volumen behalten und so eine erhöhte Druckstabilität gewährleisten kann. Geeignete gekräuselte Fasern sind 2-dimensional gekräuselte Fasern wie beispielsweise die "Grisuten"-Polyesterfasern der Firma Märkische Faser GmbH in Premnitz. Besonders geeignet sind 3-dimensional gekräuselte Fasern, insbesondere 3-dimensional gekräuselte Bindefasern (z.B. spiral gekräuselte Fasern), weil sie gegenüber der 2-dimensional gekräuselten Faser ein erhöhtes Rückerholungsvermögen aufweisen. Die 3-dimensionale Kräuselung kann schon beim Spinnprozess erzeugt werden, beispielsweise wie die "Softflex HY" von Indorama, oder entsteht bei thermischer Beaufschlagung einer Bikomponentenfaser beispielsweise mit einer side-by-side oder exzentrischen Kern-Mantel Querschnittsgeometrie, beispielsweise wie die "EMF" Faser von Huvis.

Der Anteil der gekräuselten Fasern, insbesondere der 3-dimensional gekräuselten Bindefasern, im Vertikalvliesstoff beträgt bevorzugt mindestens 20 Gew.%, beispielsweise von 20 bis 100 Gew.%, noch bevorzugter mindestens 25 Gew.%, beispielsweise von 25 bis 80 Gew.%, noch bevorzugter mindestens 30 Gew.%, beispielsweise von 30 bis 70 Gew.%, jeweils bezogen auf das Gesamtgewicht des Vertikalvliestoffs.

In einer bevorzugten Ausführungsform der Erfindung weisen die gekräuselten Fasern eine Kräuselbogenanzahl von 4 bis 25 Bögen/cm auf, bevorzugt zwischen 6 und 18 Bögen/cm, besonders bevorzugt zwischen 8 und 14 Bögen/1 cm.

In einer weiteren bevorzugten Ausführungsform der Erfindung ist der Vertikalvliesstoff thermisch verfestigt. Weiter bevorzugt ist er nicht vernadelt. An der Vernadelung ist nämlich nachteilig, dass durch sie die im Vertikalvliesstoff vorliegende vertikale Ausrichtung der Fasern gestört werden kann.

Zusätzlich zu den gekräuselten Fasern kann der Vertikalvliesstoff noch weitere, nicht gekräuselte Fasern enthalten, beispielsweise nicht gekräuselte Fasern, die Polyester, Polyolefin, Polyamid und/oder Gemische hiervon enthalten. Bevorzugt liegen die weiteren Fasern als Stapelfasern vor.

Bevorzugt sind die im Vertikalvliesstoff enthaltenen Fasern, insbesondere die Matrixfasern, Bindefasern und/oder die weiteren Fasern, Stapelfasern, bevorzugt mit einer Stapellänge zwischen 20 und 150 mm, noch bevorzugter zwischen 30 und 90 mm und insbesondere zwischen 40 und 70 mm.

Der Fasertiter der im Vertikalvliesstoff enthaltenen Fasern, insbesondere der Matrixfasern, Bindefasern und/oder der weiteren Fasern, liegt vorzugsweise im Bereich von 0,9 bis 100 dtex (g/10.000 m), noch bevorzugter liegt er zwischen 1,5 und 30 dtex, insbesondere zwischen 3 und 11 dtex.

Bevorzugt weist der Vertikalvliesstoff eine mittlere Dicke, gemessen nach DIN EN ISO 9073-2:1997-02 von mindestens 1,5 mm, beispielsweise von 1,5 mm bis 15 mm, noch bevorzugter von mindestens 2 mm, beispielsweise von 2 mm bis 10 mm, und/oder von 4 mm bis 10 mm, und/oder von 2 mm bis 5 mm und/oder von 5 mm bis 8 mm auf.

Als Schaumlage können erfindungsgemäß die verschiedensten Schäume, insbesondere Polymerschäume eingesetzt werden. Vorzugsweise basiert die Schaum lage auf Polyurethanschaum, beispielsweise Polyetherpolurethan- oder Polyesterpolyurethanschaum Polyetheresterpolyurethanschaum, Polyvinylacetatschaum, Polyvinylalkoholschaum oder auf Mischungen dieser Schäume. Der Begriff "basierend auf" meint dabei mehr als 50 Gew.%, noch bevorzugter mehr als 70 Gew.%, insbesondere mehr als 90 Gew.% bezogen auf das Gesamtgewicht der Schaumlage.

Besonders bevorzugt basiert die Schaumlage auf einem hydrophilen Polymerschaum, das heißt einem Schaum, mit einer Einsinkzeit eines Wassertropfens von weniger als 1 Minute, bevorzugt von weniger als 40 Sekunden, noch bevorzugter von weniger als 10 Sekunden, noch bevorzugter von weniger als 1 Sekunde. Ebenfalls bevorzugt ist ein Polymerschaum, der aus hydrophilen Polymeren, bevorzugt hydrophilen Polyurethanen, insbesondere hydrophilen Polyurethanen wie in der WO2018/007093 beschrieben, gefertigt ist. Hydrophile Polymerschäume haben die Fähigkeit, Flüssigkeiten zu absorbieren und zu speichern. Gegenüber hydrophoben Polymerschäumen haben hydrophile Polymerschäume somit den Vorteil, dass sie Flüssigkeit schnell von der Hautoberfläche absorbieren und so positiv zum Mikroklima auf der Haut beitragen. Dies ist im erfindungsgemäßen System besonders vorteilhaft, da die Schaumlage lediglich durch die adhäsive Schicht von der Haut getrennt wird. Ganz besonders bevorzugt ist ein Polyurethanschaum, da dieser eine hohe Hydrophilie mit einer guten Elastizität und Retention verbindet.

In einer bevorzugten Ausführungsform liegt die Schaumlage mittels eines Bindemittels mit dem textilen Flächengebilde verbunden vor. Bevorzugte Bindemittel sind adhäsive Vliesstoffe, adhäsive Gitter und/oder Schmelzkleber. Die Bindemittel können Co-Polyamid, Co-Polyester, Polyolefin, Polyvinylalkohol (PVA), Ethylen-Vinylacetat (EVA), thermoplastisches Polyurethan (TPU), Polycaprolacton, Terpolymere und/oder Mischungen hiervon enthalten. Bevorzugt enthält das Bindemittel die vorgenannten Polymere in einer Menge von mehr als 50 Gew.%, besonders bevorzugt von mehr als 70 Gew.%, insbesondere mehr als 90 Gew.%, jeweils bezogen auf das Gesamtgewicht des Bindemittels. Mit diesen Bindemitteln kann eine gute Verbindung zwischen Schaum lage und dem textilen Flächengebilde erzielt werden, ohne den Feuchtigkeitstransport negativ zu beeinflussen. Besonders bevorzugte Schmelzkleber sind thermoplastische Schmelzkleber, insbesondere Schmelzkleber auf Basis von Polycaprolacton. Hieran ist vorteilhaft, dass eine gute Haftung erzielt werden kann, ohne die Flexibilität der Hautauflage und so die Anpassbarkeit an Körperkonturen zu beeinträchtigen.

Das Auftragen des Schmelzklebers kann mit den verschiedensten dem Fachmann bekannten Verfahren erfolgen, beispielsweise mittels Streuen, Sprühen, Düsenauftrag oder Schlitzdüsenauftrag. Besonders bevorzugt ist Streuen, da hierdurch punktuelle Verbindungen geschaffen werden, die eine flexible und wasserdampfdurchlässige Verbindung ermöglichen.

In einer besonders bevorzugten Ausführungsform der Erfindung ist das Bindemittel punktuell zwischen Schaumlage und textilem Flächengebilde aufgebracht. Dies ist vorteilhaft, da hierdurch eine gute Wasserdampfdurchlässigkeit und Flexibilität aufrechterhalten werden kann.

Bevorzugt weist die Schaumlage eine mittlere Dicke, gemessen mit Hilfe eines kalibrierten Dickenmessgerätes von mindestens 0,3 mm, beispielsweise von 0,3 mm bis 12 mm, noch bevorzugter von mindestens 0,4 mm, beispielsweise von 0,4 mm bis 12 mm, noch bevorzugter von 0,5 mm bis 10 mm, noch bevorzugter von 1 mm bis 8 mm und insbesondere von 1 mm bis 7 mm auf.

Erfindungsgemäß weist die Hautauflage eine adhäsive Schicht auf. Diese kann die verschiedensten dem Fachmann bekannten Materialien enthalten, sofern diese sowohl hautverträglich sind als auch eine ausreichende Haftwirkung erzielen, um ein Verrutschen oder Ablösen der Hautauflage zu verhindern. Geeignet sind beispielsweise Silikon, Acryl und/oder Acrylat basierte Adhäsive, wobei unter "basierend" mehr als 50 Gew.% zu verstehen ist, noch bevorzugter mehr als 70 Gew.%, insbesondere mehr als 90 Gew.%, bezogen auf das Gesamtgewicht der adhäsiven Schicht. Ebenfalls geeignet sind Hydrogele, Hydrokolloide, Polyurethangele und/oder Gummi basierte Adhäsive. Vorzugsweise ist die adhäsive Schicht eine Silikon aufweisende Schicht, vorzugsweise eine Schicht, die mehr als 50 Gew.%, noch bevorzugter mehr als 70 Gew.%, insbesondere mehr als 90 Gew.% Silikon, bezogen auf die Gesamtmenge der adhäsiven Schicht, enthält. Besonders bevorzugt ist eine Silikon aufweisende adhäsive Schicht, wie sie beispielsweise in der WO2018/007093 A1 beschrieben ist. An Silikon ist vorteilhaft, dass es ein sehr weiches Adhäsiv ist, das sich gut an die Körperkontur anpassen kann. Seine Weichheit und Elastizität ermöglichen zusätzlich eine gute Aufnahme entstehender Scherkräfte. Des Weiteren ist Silikon ein sehr hautfreundliches Adhäsiv, welches sich gut und schonend von der Haut ablösen lässt ohne Hautzellen zu zerstören oder Schmerzen zu verursachen.

Die adhäsive Schicht kann die gesamte Oberfläche der Schaumlage bedecken. Hieran ist vorteilhaft, dass eine hohe Anhaftung zur Haut erzielt wird und so ein Verrutschen oder Ablösen der Hautauflage, z.B. beim Lagern von Patienten verhindert wird. Alternativ kann die adhäsive Schicht lediglich einen Teil der Schaumlage bedecken und in Form von einem Muster, beispielsweise Wellen, Punkten, Streifen und/oder als gitterförmiges Muster vorliegen. Hieran ist vorteilhaft, dass die Adhäsion auf der Haut selektiv gesteuert werden kann. Dies ist beispielsweise für besonders empfindliche Haut, wie sie beispielsweise bei älteren Patienten zu finden ist, (sogenannte Pergamenthaut) von Vorteil. Bevorzugt beträgt der Bedeckungsgrad der Schaum lage mit der adhäsiven Schicht, weniger als 90%, beispielsweise zwischen 50% und 90%, noch bevorzugter zwischen 60% und 80%. Der Bedeckungsgrad kann mittels optischer Auswertung ermittelt werden.

In einer bevorzugten Ausführungsform der Erfindung weist die adhäsive Schicht eine Dicke von weniger als 200 µm auf und/oder liegt in einer Auftragsmenge von weniger als 200 g/m² vor. Dies kann beispielsweise mit der in der WO 2018/007093 beschriebenen Vorgehensweise realisiert werden. Hieran ist vorteilhaft, dass aufgrund der geringen Dicke und ggf. Teilbelegung der adhäsiven Schicht eine höhere Wasserdampfdurchlässigkeit und niedrigere Schälkräfte realisiert werden können, wobei letztere ein hautschonendes Entfernen der Auflage ermöglichen.

In einer weiteren bevorzugten Ausführungsform weist die Hautauflage eine Einsinkzeit für Wassertropfen von weniger als 60 Sekunden, noch bevorzugter von weniger als 30 Sekunden, noch bevorzugter von weniger als 15 Sekunden auf. Hieran ist vorteilhaft, dass entstehende Flüssigkeit schnell von der Haut entfernt und so das Ansammeln von Flüssigkeit verhindert wird.

In einer weiteren bevorzugten Ausführungsform der Erfindung weist die Hautauflage eine Dicke, gemessen nach DIN EN ISO 9073-2:1997-02 von 3 bis 20 mm, noch bevorzugter von 5 bis 13 mm, noch bevorzugter von 8 bis 10 mm auf. Es wurde gefunden, dass bei diesen Dicken eine gute Druckverteilung erfolgt und die Hautauflage gleichzeitig dünn genug ist, sodass sie beispielsweise beim Wenden des Patienten nicht behindert und sich auch nicht leicht ablöst.

Für manche Anwendungszwecke ist es vorteilhaft, wenn die Hautauflage als Rollenware ausgestaltet ist. Hierdurch kann der Anwender die gewünschte Form und Größe je nach Bedarf frei auswählen. Alternativ kann die Hautauflage bereits in der für den Anwendungszweck konfektionierten Form vorliegen, beispielsweise als Fersen-, Hinterkopf- und/oder Sakralschutzauflage.

Es ist denkbar, dass die Hautauflage eine Barrierelage, beispielsweise einen Barrierefilm aufweist. Dieser besteht vorzugsweise aus Polyurethan oder Polyester oder Mischungen hiervon. Durch die Barrierelage kann die Haut vor Flüssigkeiten und aggressiven Körperausscheidungen geschützt werden. Zweckmäßigerweise ist die Barrierelage wasserdampfdurchlässig, insbesondere weist sie vorzugsweise eine Wasserdampfdurchlässigkeit (MVTR Wert) gemessen nach DIN EN 13726-2:2002-06 von mindestens 1000 g/(24 h m²), bevorzugt von mindestens 5000 g/(24 h m²), noch bevorzugter von mindestens 8000 g/(24 h m²) auf. Hieran ist vorteilhaft, dass eine Sammlung von Feuchtigkeit zwischen Haut und Hautauflage, die zu einer erhöhten Reibung auf der Haut führen würde, verhindert werden kann.

In einer bevorzugten Ausführungsform der Erfindung ist die Barrierelage auf der der Schaumlage abgewandten Seite des textilen Flächengebildes angeordnet. Weiter bevorzugt weist die Barrierelage eine Dicke, gemessen nach DIN ISO 23529:2010 von 10 bis 70 µm, besonders bevorzugt von 20 - 40 µm auf.

Die erfindungsgemäße Hautauflage eignet sich hervorragend zur Verhinderung und/oder der Vorbeugung der Entstehung von Druckgeschwüren bei temporär oder permanent immobilisierten Patienten. Bevorzugte Verwendungen umfassen den Schutz von knöchrigen Körperregionen wie beispielsweise Ferse, Hinterkopf und Sakralbereich vor Druckgeschwüren.

Ein weiterer Gegenstand der Erfindung ist ein Verfahren zur Herstellung der erfindungsgemäßen Hautauflage umfassend folgende Schritte
- Bereitstellen einer Schaumlage;
- Aufbringen einer für den Hautkontakt vorgesehenen, adhäsiven Schicht auf die Schaumlage;
- Aufbringen eines textilen Flächengebildes auf die der adhäsiven Schicht abgewandte Seite der Schaumlage, wobei das textile Flächengebilde ein Vertikalvliesstoff ist;
- Verbinden von Schaumlage und textilem Flächengebilde.

In einer bevorzugten Ausführungsform erfolgt das Verbinden von Schaumlage und textilem Flächengebilde mittels eines Bindemittels. Dieses Verbinden erfolgt bevorzugt durch Temperatur und Druck.

Die in Bezug auf die erfindungsgemäße Hautauflage diskutierten bevorzugten Ausführungsformen stellen auch im Bezug auf das erfindungsgemäße Verfahren bevorzugte Ausführungsformen dar.

Messmethoden: Für die Zwecke der vorliegenden Erfindung wurden folgende Messmethoden angewandt: Grundsätzlich gilt, dass bei allen Messmethoden, bei denen Mittelwerte gebildet werden, der Fachmann die Anzahl der zur Mittelung bestimmten Werte in Abhängigkeit von deren Streuung wählt. Je größer die gefundenen Abweichungen sind, desto mehr Werte wird er in die Bestimmung einbeziehen.

**Kräuselbogenanzahl** - Unter der Kräuselbogenanzahl wird die Anzahl der halben Sinusbogen pro cm verstanden. Jede ausgeprägte Richtungsänderung wird als Bogen bezeichnet. Nur volumengebende Bogen werden gezählt. Einzelfasern werden parallel auf eine Samttafel aufgelegt. Die Fasern dürfen dabei nicht gestaucht oder verzogen werden. Zu beiden Seiten der Faser wird ein Millimeterstreifen angelegt, der im Abstand von 20 mm eine waagrechte, linienförmige Markierung aufweist. Je 1 Messingblech auf die Markierungslinien legen (Messlänge genau definiert). Anzahl der halben Sinusbogen auf eine gekräuselte Faserlänge von 20 mm unter dem Stereomikroskop bei 12-facher Vergrößerung zählen und als Kräuselbogenzahl in Bögen/cm notieren. Jede ausgeprägte Richtungsänderung wird als Bogen bezeichnet. Spiralgekräuselte Fasern werden im eingespannten Zustand während der Einkräuselung abgelesen.

**Einsinkzeit für Wassertropfen** - Die Zeit, die ein Wassertropfen benötigt, um vollständig in die Hautauflage einzusinken wird wie folgt gemessen. Die Hautauflage wird flach mit der Silikon-beschichteten Wundkontaktschicht ausgelegt. Mit einer Pipette wird ein Wassertropfen aufgebracht und die Zeit gestoppt, die der Wassertropfen zum vollständigen Einsinken in die Hautauflage benötigt. Sollte der Wassertropfen innerhalb von 3 Minuten nicht vollständig eingesunken sein, ist die Einsinksinkzeit mit 180 Sekunden anzunehmen.

**Die Dicke der adhäsiven Schicht** - Die Dicke der adhäsiven Schicht wird mittels Rasterelektronenmikroskopie mit einer Beschleunigungsspannung von 20 kV durchgeführt. Um Aufladungseffekte und daraus resultierende Messfehler zu vermeiden, werden die Proben vor der REM-Untersuchung mit Gold besputtert. Dies geschieht bei einem Argongasdruck von 0,1 mbar bei 30 mA Sputterstrom in einem Abstand von 10 cm. Die Sputterzeit beträgt 300 Sekunden. Es wird eine fiktive Referenzoberfläche dadurch generiert, dass ein beidseitig Polyethylen-beschichtetes Papier mit einem Flächengewicht von 120 g/m² auf die adhäsive Schicht aufgelegt wird. Die Dicke wird als Distanz zwischen Unterseite des Papiers und der tiefsten, Adhäsiv enthaltenden Stelle an der jeweiligen Messstelle bestimmt. Die Auswertung erfolgt mittels REM im Querschnitt. Falls es den Kontrast zwischen Adhäsiv und Schaumlage erhöht, wird ein Rückstreudetektor verwendet. Die Dicke wird an mindestens 10 Stellen gleichmäßig verteilt über einen Bereich von mindestens 2 mm gemessen und der Mittelwert bestimmt. Um eine Verfälschung durch nachträgliches Eindringen von Adhäsiv in die Schaumlage beim Bilden der Querschnittsfläche zu vermeiden, wird der Schnitt senkrecht von der dem Adhäsiv abgewandten Seite der Schaumlage ausgeführt.

**Dicke der Schaumlage** - Die Schaumdicke wird an allen vier Ecken eines 10 x 10 cm Musters mit Hilfe eines kalibrierten Dickenmessgerätes gemessen. Bei Messung ist darauf zu achten, dass der Schaum nicht komprimiert wird. Die Messergebnisse sind mit zwei Nachkommastellen zu dokumentieren. Die mittlere Schaumdicke ergibt sich aus dem Mittelwert der vier Messungen.

**Dicke textiles Flächengebilde** - Die Dicke des textilen Flächengebildes wird gemessen nach DIN EN ISO 9073 Teil 2.

Die Erfindung wird im Folgenden anhand mehrerer Beispiele näher erläutert.

### Beispiel 1: Herstellung einer erfindungsgemäßen Hautauflage mit einem Vertikalvliesstoff als textiles Flächengebilde

Es wird eine Schaumlage aus Polyurethan (Dicke 3 mm) entsprechend dem in der WO 2018/007093 A1 beschriebenen Verfahren mit einer sehr dünnen Silikonschicht (20 g/m² 50% Bedeckung) versehen. Anschließend wird ein Vertikalvliesstoff mit einer Dichte von 20 kg/ m³ bestehend aus 60% Polyestermatrixfasern 40% spiral gekräuselter Bindefaser ("EMF" Huvis) nach dem Verfahren beschrieben in der CN104805597 in 8 mm Dicke hergestellt, auf die Schaumlage aufgebracht und mit Hilfe von Schmelzklebepulver thermisch (140°C, 6 m/min) in einer Laminieranlage verbunden.

Die hierbei erhaltene Hautauflage weist die in der nachfolgenden Tabelle dargestellten Eigenschaften auf.

| | Gesamtdicke [mm] | Dicke Schaumlage [mm] | Dicke textiles Flächengebilde [mm] | Dicke adhäsive Schicht [µm] | Einsinkzeit Wassertropfen [s] |
|---|---|---|---|---|---|
| Hautauflage | 10 | 3 | 8 | 40 | 14 |
| Mepilex Border^{®} | 4 | 1,6 | 0,2 + 2,2 | 300 | > 180 |
| Allevyn Life ^{®} | 7 | 1,7 | 3,7 + 1,6 | 250 | 24 |

### Beispiel 2: Bestimmung Kraft-Dehnungsverhalten der Hautauflage aus Beispiel 1

Das Kraft - Dehnungsverhalten der Hautauflage aus Beispiel 1 wird mit Hilfe einer Zwick Zugprüfmaschine wie folgt gemessen. Aus der Hautauflage wird eine Probe mit einem Durchmesser von 25 mm gestanzt. Die Proben werden in die Zugprüfmaschine eingelegt und mit einer Metallplatte (Durchmesser ebenfalls 25 mm) mit einer Vorkraft von 0,5 N angefahren, um einen definierten Startpunkt zu gewährleisten. Die Probe wird anschließend mit einer Kraft von 15 N (Geschwindigkeit 5 mm/s) für 15 Minuten belastet. Nach Entlastung wird die Probe erneut kurzzeitig mit 25 N belastet.

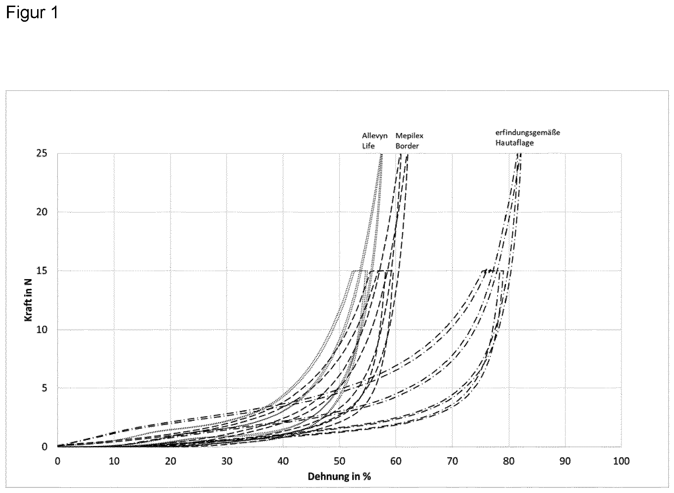

In Figur 1 wird das Kraft-Dehnungsverhalten der Hautauflage aus Beispiel 1 illustriert (gestrichelte Linie) und mit zwei kommerziell verfügbaren Hautauflagen Mepilex^{®} Border (gepunktete Linie), Allevyn Life^{®} (Punkt-Strich-Linie) verglichen. Es zeigt sich, dass die erfindungsgemäße Hautauflage eine höhere Dehnung aufweist, als die beiden kommerziellen Hautauflagen. Ausschlaggebend ist der Punkt der Kurve, in dem der lineare Anstieg der Kraft mit der Dehnung in einen exponentiellen Anstieg übergeht. Ab diesem Punkt ist die Auflage soweit komprimiert, dass ein Einsinken nur bedingt möglich ist, und durch die vorhandene Komprimierung die Steifigkeit stark ansteigt. Man sieht deutlich, dass für die erfindungsgemäße Hautauflage bei gleicher Gewichtskraft eine höhere Dehnung und somit höhere Eindringtiefe erreicht wird. Bei den kommerziellen Auflagen Mepilex^{®} Border und Allevyn Life^{®} liegt die Dehnung bei einer Gewichtskraft von 13 N bei 54% beziehungsweise 51%, wohingegen bei der erfindungsgemäßen Hautauflage die Dehnung bei ca. 76% liegt. Dies ist für die Anwendung zur Dekubitusprävention vorteilhaft, da durch dieses Verhalten die Auflageoberfläche für das zu schützende Körperteil erhöht und so der Druck über eine größere Fläche verteilt wird, wodurch Belastungsspitzen reduziert werden.

### Beispiel 3: Analyse der im Gewebe auftretenden Kräfte bei Verwendung der erfindungsgemäßen Hautauflage

Zur Bewertung der erfindungsgemäßen Hautauflage wurde die Finite-Elemente Modellierung (FEM) herangezogen. Dekubituswunden entstehen zu einem großen Teil im tieferliegen Gewebe und nicht an der Hautoberfläche. Daher ist eine reine Betrachtung der Druckverteilung an der Oberfläche nicht ausreichend, um die Wirksamkeit von Hautauflagen zu beurteilen. Es wurde daher die Finite-Element Modellierung herangezogen, um die auftretenden Druckspannungen im Gewebe zu bewerten. Die Finite-Elemente-Modellierung beruht auf der Nutzung von numerischen Verfahren. Ein Festkörper wird in endliche viele Teile aufgeteilt, über deren physikalisches Verhalten wiederum das Verhalten des Gesamtkörpers berechnet werden kann.

Das zur Bewertung der erfindungsgemäßen Hautauflage gewählte Verfahren wurde adaptiert nach A. Levy, M. B-O. Frank and Amid Gefen, The biomechanical efficacy of dressings in preventing heel ulcers, in Journal of Tissue Viability (2015) 24, 1-11. Die biomechanischen Eigenschaften des Fußgewebes wurden entnommen aus Sara Behforootan, Panagiotis E. Chatzistergos, Nachiappan Chockalingam, Roozbeh Naemi, Journal of the mechanical behavior of biomedical materials 68 (2017) 287-295.

Es zeigt sich, dass bei Verwendung der erfindungsgemäßen Hautauflage bei einer Gewichtskraft von 13 N die maximalen Belastungen im Gewebe (bestimmt in Prozent als ξmax) mit 18,6% unterhalb der maximalen Belastungen bei kommerziellen Auflagen (Mepilex^{®} Border 23,3% und Allevyn Life^{®} 21,9%) liegen. Hierdurch können auftretende Druckspannungen verglichen mit den kommerziellen Hautauflagen deutlich stärker reduziert werden.

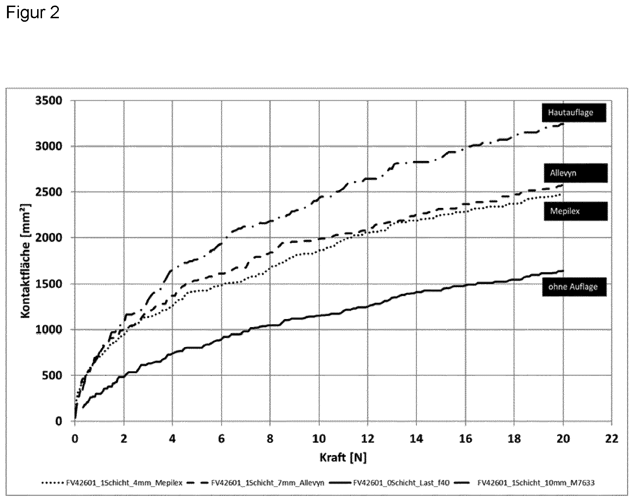

Figur 2 zeigt die Veränderung der Kontaktfläche für das zu schützende Körperteil (in der gewählten Simulation ein Fuß) mit zunehmender Gewichtskraft für eine erfindungsgemäße Hautauflage (gestrichelte Linie) verglichen mit den kommerziellen Produkten Mepilex^{®} Border (gepunktete Linie) und Allevyn Life^{®} (Punkt-Strich-Linie). Es zeigt sich, dass die erfindungsgemäße Hautauflage die Auflagefläche insbesondere auch bei hohen Kräften im Vergleich zur Verwendung keiner Auflage die Auflagefläche um 100% erhöht, wohingegen die kommerziellen Produkte Mepilex ^{®} Border und Allevyn Life ^{®} die Auflagefläche nur etwa um 50% erhöhen. Somit kann bei Verwendung der erfindungsgemäßen Hautauflage der auftretende Druck über eine größere Fläche verteilt und lokale Belastungsspitzen reduziert werden.

## Patentansprüche

1. Hautauflage umfassend eine Schaumlage und eine unmittelbar darauf angeordnete, für den Hautkontakt vorgesehene, adhäsive Schicht, wobei auf der der adhäsiven Schicht abgewandten Seite der Schaumlage ein textiles Flächengebilde angeordnet ist, **dadurch gekennzeichnet, dass** das textile Flächengebilde ein Vertikalvliesstoff ist.

2. Hautauflage nach Anspruch 1, **dadurch gekennzeichnet, dass** der Vertikalvliesstoff Matrixfasern, vorzugsweise ausgewählt aus Polyester-, Polyolefin-, Polyamid, Zellulose- Fasern und/oder Gemische hiervon, enthält.

3. Hautauflage nach Anspruch 1 oder 2, **dadurch gekennzeichnet, dass** der Anteil der Matrixfasern im Vertikalvliesstoff, bezogen auf das Gesamtgewicht des Vertikalvliestoffs, mindestens 20 Gew.% beträgt.

4. Hautauflage nach einem oder mehreren der vorangehenden Ansprüche, **dadurch gekennzeichnet, dass** der Vertikalvliesstoff Bindefasern enthält, vorzugsweise Bindefasern, die Polyolefin-, Polyester-, PolyamidFasern und/oder Gemische hiervon enthalten und/oder Bikomponentenfasern enthält, insbesondere Bikomponentenfasern, die Polyolefin, Polyester, Polyamid, Ethylen-Vinylacetat, Ethylen-Methylacrylat, Polybutylenterephtalat, Ethylenacrylat, Ethylenvinylacetat Co-Polymer, Acrylnitril und/oder Gemische hiervon als außenliegende Faserkomponente enthalten.

5. Hautauflage nach einem oder mehreren der vorangehenden Ansprüche, **dadurch gekennzeichnet, dass** der Vertikalvliesstoff gekräuselte Fasern, insbesondere gekräuselte Bindefasern, enthält.

6. Hautauflage nach einem oder mehreren der vorangehenden Ansprüche, **dadurch gekennzeichnet, dass** der Vertikalvliesstoff thermisch verfestigt und vorzugsweise nicht vernadelt ist.

7. Hautauflage nach einem oder mehreren der vorangehenden Ansprüche, **dadurch gekennzeichnet, dass** der Vertikalvliesstoff eine mittlere Dicke, gemessen nach DIN EN ISO 9073-2:1997-02 von mindestens 1,5 mm aufweist.

8. Hautauflage nach einem oder mehreren der vorangehenden Ansprüche, **dadurch gekennzeichnet, dass** die Schaumlage auf hydrophilen Polymerschäumen, insbesondere Polyurethanschäumen, basiert.

9. Hautauflage nach einem oder mehreren der vorangehenden Ansprüche, **dadurch gekennzeichnet, dass** die Schaumlage mittels eines Bindemittels, insbesondere adhäsiven Vliesstoffen, adhäsiven Gittern und/oder Schmelzkleber, mit dem textilen Flächengebilde verbunden vorliegt.

10. Hautauflage nach einem oder mehreren der vorangehenden Ansprüche, **dadurch gekennzeichnet, dass** die adhäsive Schicht eine Silikon aufweisende, insbesondere im Wesentlichen aus Silikon bestehende, adhäsive Schicht ist.

11. Hautauflage nach einem oder mehreren der vorangehenden Ansprüche, **dadurch gekennzeichnet, dass** die adhäsive Schicht eine Dicke von weniger als 200 µm aufweist und/oder in einer Auftragsmenge von weniger als 200 g/m² vorliegt.

12. Hautauflage nach einem oder mehreren der vorangehenden Ansprüche, **gekennzeichnet durch** eine Dicke, gemessen nach DIN EN ISO 9073-2:1997-02 von 3 bis 15 mm.

13. Hautauflage nach einem oder mehreren der vorangehenden Ansprüche, **dadurch gekennzeichnet, dass** sie als Rollenware ausgestaltet ist.

14. Verfahren zur Herstellung einer Hautauflage nach einem oder mehreren der Ansprüche 1 - 13 umfassend folgende Schritte
- Bereitstellen einer Schaumlage;
- Aufbringen einer für den Hautkontakt vorgesehenen, adhäsiven Schicht auf die Schaumlage;
- Aufbringen eines textilen Flächengebildes auf die der adhäsiven Schicht abgewandte Seite der Schaumlage, wobei das textile Flächengebilde ein Vertikalvliesstoff ist;
- Verbinden von Schaumlage und textilem Flächengebilde.

## Claims

1. Dermal dressing comprising a foam ply and an adhesive layer disposed directly thereon and intended for skin contact, where a textile fabric is disposed on the side of the foam ply facing away from the adhesive layer, **characterized in that** the textile fabric is a vertically-lapped nonwoven fabric.

2. Dermal dressing according to Claim 1, **characterized in that** the vertically-lapped nonwoven fabric comprises matrix fibres, preferably selected from polyester, polyolefin, polyamide and cellulose fibres and/or mixtures thereof.

3. Dermal dressing according to Claim 1 or 2, **characterized in that** the proportion of the matrix fibres in the vertically-lapped nonwoven fabric, based on the total weight of the vertically-lapped nonwoven fabric, is at least 20% by weight.

4. Dermal dressing according to one or more of the preceding claims, **characterized in that** the vertically-lapped nonwoven fabric comprises binding fibres, preferably binding fibres which comprise polyolefin, polyester, polyamide fibres and/or mixtures thereof, and/or comprises bicomponent fibres, more particularly bicomponent fibres which comprise polyolefin, polyester, polyamide, ethylene-vinyl acetate, ethylene-methyl acrylate, polybutylene terephthalate, ethylene acrylate, ethylene-vinyl acetate copolymer, acrylonitrile and/or mixtures thereof as outer fibre component.

5. Dermal dressing according to one or more of the preceding claims, **characterized in that** the vertically-lapped nonwoven fabric comprises crimped fibres, more particularly crimped binding fibres.

6. Dermal dressing according to one or more of the preceding claims, **characterized in that** the vertically-lapped nonwoven fabric is thermally consolidated and preferably not needled.

7. Dermal dressing according to one or more of the preceding claims, **characterized in that** the vertically-lapped nonwoven fabric has an average thickness, measured in accordance with DIN EN ISO 9073-2:1997-02, of at least 1.5 mm.

8. Dermal dressing according to one or more of the preceding claims, **characterized in that** the foam ply is based on hydrophilic polymer foams, more particularly polyurethane foams.

9. Dermal dressing according to one or more of the preceding claims, **characterized in that** the foam ply is joined to the textile fabric by means of a binder, more particularly adhesive nonwoven fabrics, adhesive lattices and/or hotmelt adhesive.

10. Dermal dressing according to one or more of the preceding claims, **characterized in that** the adhesive layer is an adhesive layer comprising silicone, more particularly consisting substantially of silicone.

11. Dermal dressing according to one or more of the preceding claims, **characterized in that** the adhesive layer has a thickness of less than 200 µm and/or is present in an application quantity of less than 200 g/m².

12. Dermal dressing according to one or more of the preceding claims, **characterized by** a thickness, measured in accordance with DIN EN ISO 9073-2:1997-02, of 3 to 15 mm.

13. Dermal dressing according to one or more of the preceding claims, **characterized in that** it is configured as roll product.

14. Method for producing a dermal dressing according to one or more of Claims 1 - 13, comprising steps as follows:
- providing a foam ply;
- applying an adhesive layer, intended for skin contact, to the foam ply;
- applying a textile fabric to the side of the foam ply facing away from the adhesive layer, where the textile fabric is a vertically-lapped nonwoven fabric;
- joining foam ply and textile fabric.

## Revendications

1. Timbre transdermique comprenant une couche de mousse et une couche adhésive, prévue pour contact avec la peau, disposée immédiatement par-dessus, une étoffe textile étant disposée sur la face de la couche de mousse opposée à la couche adhésive, **caractérisé en ce que** l'étoffe textile est un non-tissé vertical.

2. Timbre transdermique selon la revendication 1, **caractérisé en ce que** le non-tissé vertical contient des fibres de matrice, de préférence choisies parmi les fibres de polyester, de polyoléfine, de polyamide, de cellulose et/ou les mélanges de celles-ci.

3. Timbre transdermique selon l'une quelconque des revendications 1 ou 2, **caractérisé en ce que** la proportion des fibres de matrice dans le non-tissé vertical, rapportée au poids total du non-tissé vertical, est d'au moins 20 % en poids.

4. Timbre transdermique selon l'une ou plusieurs quelconques des revendications précédentes, **caractérisé en ce que** le non-tissé vertical contient des fibres de liaison, de préférence des fibres de liaison qui contiennent des fibres de polyoléfine, de polyester, de polyamide et/ou des mélanges de celles-ci et/ou contient des fibres bicomposants, en particulier des fibres bicomposants qui contiennent une polyoléfine, un polyester, un polyamide, un éthylène-acétate de vinyle, un éthylène-acrylate de méthyle, un poly(téréphtalate de butylène), un acrylate d'éthylène, un copolymère éthylène-acétate de vinyle, un acrylonitrile et/ou des mélanges de ceux-ci, en tant que composant fibre extérieur.

5. Timbre transdermique selon l'une ou plusieurs quelconques des revendications précédentes, **caractérisé en ce que** le non-tissé vertical contient des fibres frisées, en particulier des fibres de liaison frisées.

6. Timbre transdermique selon l'une ou plusieurs quelconques des revendications précédentes, **caractérisé en ce que** le non-tissé vertical est lié par voie thermique et de préférence n'est pas aiguilleté.

7. Timbre transdermique selon l'une ou plusieurs quelconques des revendications précédentes, **caractérisé en ce que** le non-tissé vertical présente une épaisseur moyenne, mesurée selon DIN EN ISO 9073-2:1997-02 d'au moins 1,5 mm.

8. Timbre transdermique selon l'une ou plusieurs quelconques des revendications précédentes, **caractérisé en ce que** la couche de mousse est à base de mousses polymères hydrophiles, en particulier de mousses de polyuréthane.

9. Timbre transdermique selon l'une ou plusieurs quelconques des revendications précédentes, **caractérisé en ce que** la couche de mousse se présente assemblée à l'étoffe textile à l'aide d'un liant, en particulier des non-tissés adhésifs, des grilles adhésives et/ou des adhésifs fusibles.

10. Timbre transdermique selon l'une ou plusieurs quelconques des revendications précédentes, **caractérisé en ce que** la couche adhésive est une couche adhésive contenant une silicone, en particulier constituée pour l'essentiel de silicone.

11. Timbre transdermique selon l'une ou plusieurs quelconques des revendications précédentes, **caractérisé en ce que** la couche adhésive présente une épaisseur inférieure à 200 µm et/ou est présente selon une masse surfacique inférieure à 200 g/m².

12. Timbre transdermique selon l'une ou plusieurs quelconques des revendications précédentes, **caractérisé par** une épaisseur, mesurée selon DIN EN ISO 9073-2:1997-02, de 3 à 15 mm.

13. Timbre transdermique selon l'une ou plusieurs quelconques des revendications précédentes, **caractérisé en ce qu'**il se présente en rouleau.

14. Procédé de fabrication d'un timbre transdermique selon l'une ou plusieurs quelconques des revendications 1 à 13, comprenant les étapes suivantes
- fourniture d'une couche de mousse ;
- application d'une couche adhésive, prévue pour contact avec la peau, sur la couche de mousse ;
- application d'une étoffe textile sur la face de la couche de mousse opposée à la couche adhésive, l'étoffe textile étant un non-tissé vertical ;
- assemblage de la couche de mousse et de l'étoffe textile.
